**Europäisches Patentamt**

**European Patent Office**

**Office européen des brevets**

(19)

(11) Veröffentlichungsnummer : **0 027 966**
**B1**

(12) # EUROPÄISCHE PATENTSCHRIFT

(45) Veröffentlichungstag der Patentschrift :
24.08.83

(21) Anmeldenummer : **80106310.8**

(22) Anmeldetag : **16.10.80**

(51) Int. Cl.³ : **A 61 B   6/02**, G 01 T   1/164,
G 01 T   1/20

(54) **Strahlendiagnostikgerät für die Erzeugung von Schichtbildern eines Aufnahmeobjektes.**

(30) Priorität : **24.10.79 DE 2943027**

(43) Veröffentlichungstag der Anmeldung :
**06.05.81 Patentblatt 81/18**

(45) Bekanntmachung des Hinweises auf die Patenterteilung : **24.08.83 Patentblatt 83/34**

(84) Benannte Vertragsstaaten :
**FR GB IT SE**

(56) Entgegenhaltungen :
**FR A 2 372 439**
**GB A 2 005 405**
**GB A 2 005 953**
**GB A 2 030 422**
**US A 4 153 839**

(73) Patentinhaber : **SIEMENS AKTIENGESELLSCHAFT**
**Berlin und München Wittelsbacherplatz 2**
**D-8000 München 2 (DE)**

(72) Erfinder : **Krumme, Hans Jochen, Dipl.-Ing.**
**Amselweg 6**
**D-8521 Uttenreuth (DE)**
Erfinder : **Schmidt, Martin**
**Eltersdorferstrasse 1**
**D-8520 Erlangen (DE)**
Erfinder : **Schmitt, Günter**
**Forststrasse 16**
**D-8520 Erlangen (DE)**
Erfinder : **Schubert, Wolfgang, Dr., Dipl.-Chem.**
**Fontanestrasse 3**
**D-8520 Erlangen (DE)**

EP 0 027 966 B1

## Strahlendiagnostikgerät für die Erzeugung von Schichtbildern eines Aufnahmeobjektes

Die Erfindung bezieht sich auf ein Strahlendiagnostikgerät für die Erzeugung von Schichtbildern eines Aufnahmeobjektes mit einer Lagerstatt, mit einer Meßanordnung zur Durchstrahlung des Aufnahmeobjektes aus verschiedenen Richtungen, bestehend aus einer Strahlenquelle, welche ein die zu untersuchende Schicht durchdringendes Strahlenbündel aussendet, dessen Abmessung senkrecht zur Schichtebene gleich der Schichtstärke ist, und einem Strahlenempfänger, welcher der gemessenen Strahlenintensität entsprechende elektrische Ausgangssignale liefert und mit einem am Strahlenempfänger angeschlossenen Rechner zur Berechnung der Schwächungswerte bestimmter Bildpunkte der durchstrahlten Körperschicht aus den Ausgangssignalen des Strahlenempfängers, bei dem der Strahlenempfänger aus einer Detektorreihe besteht und ein Kollimator mit je einem Kollimatorschacht für jeden Detektor vorhanden ist und bei dem jeder Detektor in dem ihm zugeordneten Kollimatorschacht an einem den Schacht begrenzenden Blech aus strahlenabsorbierendem Material angeordnet ist und aus einem Szintillationskristall und einem optisch damit in Kontakt stehenden lichtelektrischen Wandler besteht, der am Blech befestigt ist.

Ein Strahlendiagnostikgerät dieser Art, ein sogenannter Computer-Tomograph, ist in der DE-A-28 40 965 beschrieben. Bei diesem Strahlendiagnostikgerät ist ein optimaler Schutz der Detektoren des Strahlenempfängers gegen Streustrahlung gegeben, weil die Detektoren in den Kollimatorschächten liegen und damit durch die Kollimatorbleche vor Streustrahlung geschützt werden. Ein einzelner Detektor kann dabei leicht ausgewechselt werden, indem das Blech, an dem er befestigt ist, aus dem Kollimator herausgezogen und gegen ein anderes Blech mit einem anderen Detektor ausgetauscht wird.

Der Erfindung liegt die Aufgabe zugrunde, ein Strahlendiagnostikgerät der eingangs genannten Art so weiterzubilden, daß eine verbesserte Lichtausbeute des Szintillationslichtes erfolgt.

Diese Aufgabe ist erfindungsgemäß dadurch gelöst, daß der Szintillationskristall auf der Oberfläche des lichtelektrischen Wandlers so befestigt ist, daß dieser zwischen dem Blech und dem Szintillationskristall liegt und daß der Szintillationskristall auf seiner Oberfläche mit Ausnahme der am lichtelektrischen Wandler anliegenden Fläche mit einer optischen Abdichtung überzogen ist. Bei dem erfindungsgemäßen Strahlendiagnostikgerät ist es möglich, einen großflächigen Kontakt zwischen den lichtelektrischen Wandlern und den Szintillationskristallen der Detektoren und damit eine gute Lichtausbeute zu erzielen. Die Lichtausbeute wird noch weiter verbessert, wenn die optische Abdichtung auf ihrer am Szintillationskristall anliegenden Seite lichtreflektierend ist.

Die Erfindung ist nachfolgend anhand zweier in der Zeichnung dargestellter Ausführungsbeispiele näher erläutert. Es zeigen :

Figuren 1 und 2 zwei Ansichten der für Erfindung wesentlichen Teile eines Strahlendiagnostikgerätes nach der Erfindung und

Figur 3 eine Variante des Ausführungsbeispiels gemäß den Fig. 1 und 2.

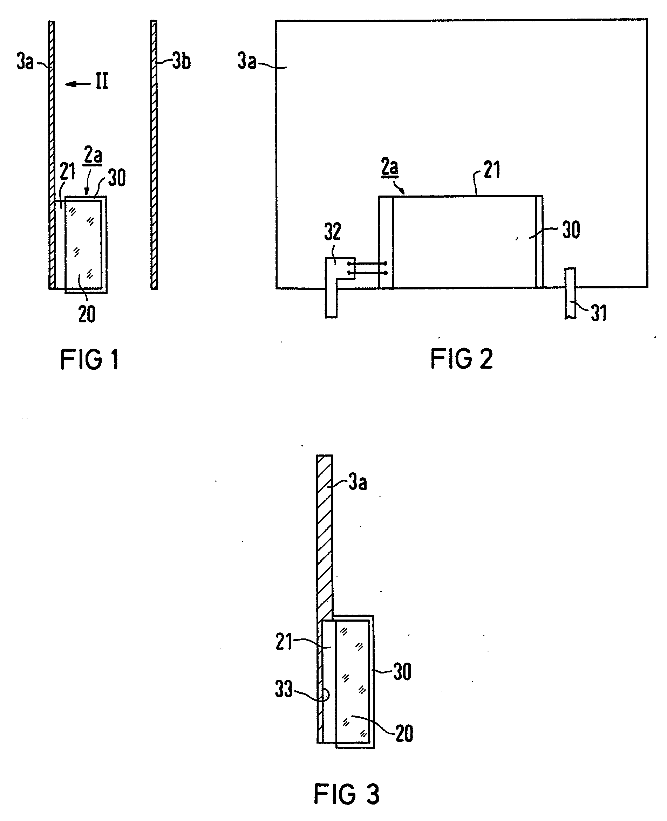

In der Fig. 1 sind zwei Bleche 3a und 3b eines Kollimators dargestellt. Die aus röntgenstrahlenabsorbierendem Material, z. B. Tantal, bestehenden Bleche 3a, 3b begrenzen dabei einen Kollimatorschacht, in dem ein Detektor 2a angeordnet ist. Der Detektor 2a besteht aus einem Szintillationskristall 20, z. B. einem CsJ/Tl-Kristall und einem lichtelektrischen Wandler 21, z. B. einer Fotodiode. Der lichtelektrische Wandler 21 ist auf dem Blech 3a festgeklebt. Auf seiner der festgeklebten Seite gegenüberliegenden Seite ist der Szintillationskristall 20 festgeklebt, so daß der lichtelektrische Wandler 21 zwischen dem Blech 3a und dem Szintillationskristall 20 liegt. Der Szintillationskristall 20 ist auf seiner Oberfläche mit Ausnahme der am lichtelektrischen Wandler 21 anliegenden Fläche mit einer optischen Abdichtung 30 überzogen, die auf ihrer am Szintillationskristall 20 anliegenden Seite lichtreflektierend ist. Die optische Abdichtung kann beispielsweise eine aufgedampfte Aluminiumschicht oder ein Reflektorlack sein. Die Lichtausbeute ist aufgrund des großflächigen Kontaktes zwischen dem lichtelektrischen Wandler 21 und dem Szintillationskristall 20 und der optischen Abdichtung 30 sehr hoch.

Aus der Fig. 2, die das Blech 3a in einer Ansicht in Richtung des Pfeiles II in Fig. 1 zeigt, geht hervor, daß das Blech 3a mit einer Kathodenzuleitung 31 versehen ist. Der eine Pol des lichtelektrischen Wandlers 21 ist somit über das Blech 3a abgeführt. Der andere Pol ist zu einem auf dem Blech 3a isoliert aufgebrachten Anodenanschluß 32 geführt.

Bei der in der Fig. 3 dargestellten Variante weist das Kollimatorblech 3a eine Aussparung 33 auf, in der der lichtelektrische Wandler 21 liegt. Er kann dort mit dem Blech 3a beispielsweise durch eine elektrisch leitende Epoxidharz-Verklebung verbunden sein. Der Szintillationskristall 20 kann mit dem lichtelektrischen Wandler durch eine transparente Epoxidharz-Verklebung verbunden sein.

### Ansprüche

1. Strahlendiagnostikgerät für die Erzeugung von Schichtbildern eines Aufnahmeobjektes mit einer Lagerstatt, mit einer Meßanordnung zur Durchstrahlung des Aufnahmeobjektes aus verschiedenen Richtungen, bestehend aus einer Strahlenquelle, welche ein die zu untersuchende Schicht durchdringendes Strahlenbündel aussendet, dessen Abmessung senkrecht zur Schichtebene gleich der Schichtstärke ist, und einem Strahlenempfänger, welcher der gemessenen Strahlenintensität entsprechende elektrische Ausgangssignale liefert und mit einem am Strahlenempfänger angeschlossenen Rechner zur Berechnung der Schwächungswerte bestimmter Bildpunkte der durchstrahlten Körperschicht aus den Ausgangssignalen des Strahlenempfängers, bei dem der Strahlenempfänger aus einer Detektorreihe besteht und ein Kollimator mit je einem Kollimatorschacht für jeden Detektor vorhanden ist und bei dem jeder Detektor in dem ihm zugeordneten Kollimatorschacht an einem den Schacht begrenzenden Blech aus strahlenabsorbierendem Material angeordnet ist und aus einem Szintillationskristall und einem optisch damit in Kontakt stehenden lichtelektrischen Wandler besteht, der am Blech befestigt ist, dadurch gekennzeichnet, daß der Szintillationskristall (20) auf der Oberfläche des lichtelektrischen Wandlers (21) so befestigt ist, daß dieser zwischen dem Blech (3a) und dem Szintillationskristall (20) liegt und daß der Szintillationskristall (20) auf seiner Oberfläche mit Ausnahme der am lichtelektrischen Wandler (21) anliegenden Fläche mit einer optischen Abdichtung (30) überzogen ist.

2. Strahlendiagnostikgerät nach Anspruch 1, dadurch gekennzeichnet, daß die optische Abdichtung (30) auf ihrer am Szintillationskristall (20) anliegenden Seite lichtreflektierend ist.

3. Strahlendiagnostikgerät nach Anspruch 1 oder 2, dadurch gekennzeichnet, daß der lichtelektrische Wandler (21) in einer Aussparung (33) des zugeordneten Bleches (3a) liegt.

**Claims**

1. Radiation ray diagnostic apparatus for the production of planigraphs of an object to be photographed, comprising a bed, a measuring arrangement to irradiate the object to be photographed from various directions, a radiation source which emits a beam which penetrates the layer to be investigated and whose dimension at right angles to the layer plane is equal to the layer thickness, and a radiation receiver which supplies electric output signals corresponding to the measured beam intensity, and a computer connected to the radiation receiver to calculate the attenuation values of specific image points of the irradiated body layer from the output signals of the radiation receiver, wherein the radiation receiver consists of a series of detectors, and a collimator is provided which possesses one collimator compartment for each detector, and each detector is arranged in the collimator compartment assigned thereto on a sheet of radiation absorbant material which defines the compartment, and a scintillation crystal and a photoelectric transducer in optical contact therewith and attached to the sheet, characterised in that the scintillation crystal (20) is attached to the surface of the photo-electric transducer (21) in such manner that the latter lies between the sheet (3a) and the scintillation crystal (20), and that with the exception of the area connected to the photoelectric transducer (21) the surface of the scintillation crystal (20) is coated with an optical seal (30).

2. Radiation diagnostic apparatus as claimed in Claim 1, characterised in that the side of the optical seal (30) connected to the scintillation crystal (20) is light-reflective.

3. Radiation diagnostic apparatus as claimed in Claim 1 or 2, characterised in that the photoelectric transducer (21) is located in a recess (33) of the assigned sheet (3a).

**Revendications**

1. Appareil de diagnostic par rayonnement pour la production de tomographies d'un objet de prise de vue, avec une surface de repos, avec un dispositif de mesure pour l'irradiation d'objet de prise de vue à partir de différentes directions, constitué par une source de rayons qui émet un faisceau de rayonnement traversant la couche à examiner et dont la dimension, perpendiculairement au plan de la couche, est égale à l'épaisseur de la couche et par un récepteur de rayonnement qui fournit des signaux électriques de sortie qui correspondent à l'intensité du rayonnement mesurée et par une calculatrice reliée au récepteur de rayonnement pour calculer les valeurs d'atténuation de certains points de l'image de la couche irradiée du corps à partir des signaux de sortie du récepteur de rayonnement, dans lequel le récepteur de rayonnement est constitué par une rangée de détecteurs, un collimateur avec respectivement un délimiteur de rayonnement étant associé à chaque détecteur, et dans lequel chaque détecteur est disposé dans le délimiteur de collimateur qui lui est associé sur une tôle limitant le délimiteur et faite avec un matériau absorbant le rayonnement et par un cristal de scintillation et par un transducteur photoélectrique optiquement en contact avec celui-ci et fixé à la tôle, caractérisé par le fait que le cristal de scintillation (20) est fixé de telle manière sur la surface du transducteur photoélectrique (21) que ce dernier se situe entre la tôle (3a) et le cristal de scintillation (20), et que le cristal de scintillation (20) est revêtu d'un moyen d'étanchéité optique (30) sur sa surface, à l'exception de la surface qui porte contre le transducteur photoélectrique (20).

2. Appareil de diagnostic par rayonnement selon la revendication 1, caractérisé par le fait que le moyen d'étanchéité (30) est réfléchissant sur son côté qui porte contre le cristal de scintillation (20).

3. Appareil de diagnostic par rayonnement selon la revendication 1 ou 2, caractérisé par le fait que le transducteur photoélectrique est situé dans une ouverture (33) ménagée dans la tôle associée (3a).

FIG 1

FIG 2

FIG 3